# EUROPEAN PATENT APPLICATION

(11) **EP 2 033 948 A1**
(43) Date of publication of application: **11.03.2009**
(21) Application number: 07075741.4
(22) Date of filing: 30.08.2007
(51) Int. Cl.: C07C 211/15, C07C 211/24, C07C 211/29, C07C 21/18, C07C 33/42, C07C 309/73, C07F 9/54, C07J 41/00

(54) **Process for preparing Estrogen-antagonistic 11beta-Fluoro-17alpha-alkylestra-1,3,5 (10)-triene-3, 17-diols having a 7alpha-(xi-Alkylamino-omega-perfluoroalkyl)alkyl side chain and alpha-Alkyl(amino)-omega-perfluoro(alkyl)alkanes and processes for their preparation**

(71) Applicant: Bayer Schering Pharma Aktiengesellschaft, 13353 Berlin (DE); AstraZeneca AB, 151 85 Södertälje (SE)
(72) Inventor: Sander, Michael. Dr., 50226 Berlin (DE); Grossbach, Danja. Dr., 12277 Berlin (DE); Dinter, Christian Dr., 13189 Berlin (DE); Hassfeld, Jorma. Dr., 10439 Berlin (DE); Voigtlaender, David. Dr., 61118 Bad Vilbel (DE)

(57) **Abstract**

The present invention relates to a new process for preparing estrogen-antagonistic 11β-fluoro-17α-alkylestra-1,3,5(10)-triene-3,17-diols of the general formula I having a 7α-(ξ-alkylamino-ω-perfluoroalkyl)alkyl side chain and to α-alkyl(amino)-ω-perfluoro(alkyl)alkanes of the general formula II, to processes for their preparation and to the intermediates required for this purpose.

## Description

The present invention relates to a new process for preparing estrogen-antagonistic 11β-fluoro-17α-alkylestra-1,3,5(10)-triene-3,17-diols of the general formula I having a 7α-(ξ-alkylamino-ω-perfluoroalkyl)alkyl side chain and to α-alkyl(amino)-ω-perfluoro(alkyl)alkanes of the general formula II, to processes for their preparation and to the intermediates required for this purpose.

More particularly, the invention relates to a process for preparing compounds of the general formula I 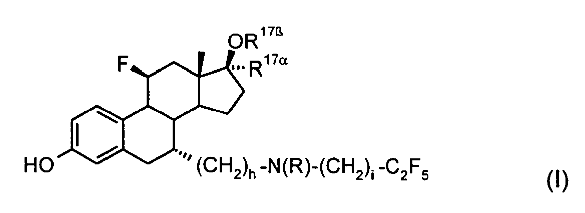 in which
R^{17α} is an alkyl group having 1 to 4 carbon atoms and which may be partially or completely fluorinated or an alkynyl group having 2 to 4 carbon atoms
R^{17β} is an hydrogen atom, an alkyl group having 1 to 4 carbon atoms or an alkanoyl group having 1 to 4 carbon atoms,
h is an integer of 1 to 6
R is an alkyl group having 1 to 3 carbon atoms and
i is an integer of 6 to 9.

The invention further relates to α-alkyl(amino)-ω-perfluoro(alkyl)alkanes of the general formula II

H(R)N-(CH₂)ᵢ-C₂F₅ (II)

in which
R is an alkyl group having 1 to 3 carbon atoms and
i is an integer of 6 to 9,
and to processes for their preparation.

The invention also relates to the intermediates required in the preparation of the α-alkyl(amino)-ω-perfluoro(alkyl)alkanes of the general formula II. These are the compounds of the general formulae VII, IX and the compounds 16b, 20, 24, 25, 26, 27 and 28.

The compounds of the general formula I are compounds with strong antiestrogenic activity. More specifically, they are estrogen antagonists which display their antiestrogenic activity owing to the competitive displacement of the natural estrogens from their receptor and/or by destabilization of the estrogen receptor. In the latter case, reference is also made to Selective Estrogen Receptor Destabilizers (SERDs). Occasionally, the same abbreviation is also understood to mean the term Selective Estrogen Receptor Downregulator. In both cases, i.e. in the case of the competitive estrogen antagonists and in the case of the SERDs, the result is suppression of the transmission of the estrogenic stimulus.
Moreover, the compounds of the general formula I are preferably pure antiestrogens, which is intended to mean that these compounds have only vanishing estrogenic residual action, if any.

These compounds of the general formula I and their preparation are described for the first time in WO 03/045972. In this case, compounds of the general formula III 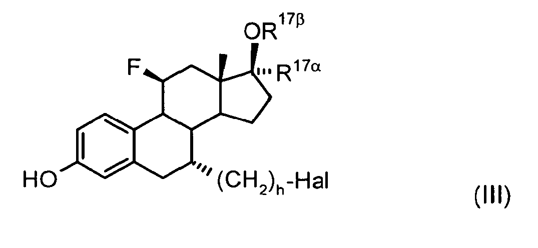 in which
R^{17α} is an alkyl group having 1 to 4 carbon atoms and which may be partially or completely fluorinated or an alkynyl group having 2 to 4 carbon atoms
R^{17β} is an hydrogen atom, an alkyl group having 1 to 4 carbon atoms or an alkanoyl group having 1 to 4 carbon atoms,
h is an integer of 1 to 6
and
Hal is a halogen atom
are reacted with an α-alkyl(amino)-ω-perfluoro(alkyl)alkane of the general formula II

H(R)N-(CH₂)ᵢ-C₂F₅ (II)

in which
R is an alkyl group having 1 to 3 carbon atoms and
i is an integer of 6 to 9
to obtain a compound of the general formula I.

In particular, R is a methyl group, i is 5 and Hal is a bromine atom, a chlorine atom or iodine atom.

The compounds of the general formula II can be prepared, inter alia, starting from α-hydroxy-ω-perfluoro(alkyl)alkanes by methods known to those skilled in the art (see Scheme 1).

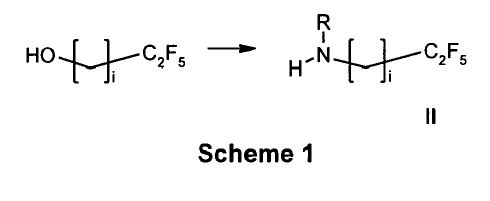

A possible route to the preparation of the α-hydroxy-ω-perfluoro(alkyl)alkanes required as starting compounds for this purpose has been described in WO 99/33855 A1 (see Scheme 2).

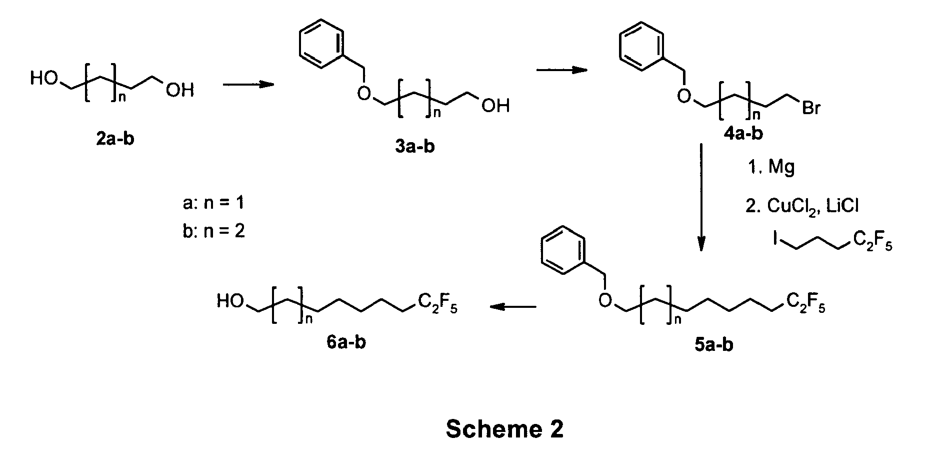

One disadvantage of the above-specified process (Scheme 2) for preparing the α-hydroxy-ω-perfluoro(alkyl)alkanes for the preparation of the compounds of the general formula II is that chromatographic purification steps of the intermediates are needed to prepare the corresponding α-hydroxy-ω-perfluoro(alkyl)alkanes in sufficient purity.
Furthermore, the reaction of the Grignard reagents prepared from compound 4a-b with 1,1,1,2,2-pentafluoro-5-iodopentane requires strict control and specialist operating procedures and while it may be possible to exercise appropriate controls at a laboratory scale, the scaling of these reactions to an industrial scale poses large technical difficulties.

Alternatively to Scheme 2, the preparation of α-hydroxy-ω-perfluoro(alkyl)alkanes is also possible via the route shown in Scheme 3, starting from α-hydroxy-ω-alkenes. **[ⁱ]**

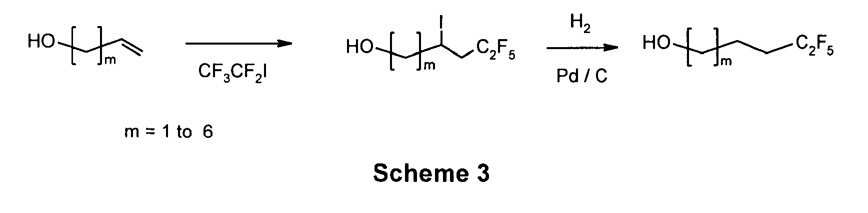

For this purpose, a perfluoroalkyl group (usually using the corresponding perfluoroalkyl iodide) is added onto a terminal double bond of an α-hydroxy-ω-alkene.
One disadvantage of the above-specified process (Scheme 3) for preparing the α-hydroxy-ω-perfluoro(alkyl)alkanes for the preparation of the compounds of the general formula II is that the starting materials to be used in this case (α-hydroxy-ω-alkenes) are commercially available in bulk amounts for syntheses on the industrial scale, i.e. in an amount on the kg scale, only in a few cases (for example allyl alcohol (m = 1) or 5-hexen-1-ol (m = 4)). A particular problem has been found in the availability of large amounts of 6-hepten-1-ol (m = 5) which, according to Scheme 3, would have to be used as a starting compound for the preparation of a specific antiestrogen of the compound of the general formula I (when i = 7). The preparation of certain amines according to Scheme 3 is therefore unsuitable for a scaleup of the laboratory synthesis to the industrial scale (kg amounts).

Furthermore, it is known that, in the addition of perfluoroalkyl halides (for example pentafluoroethyl iodide, CF₃CF₂I) for the introduction of the terminal perfluoroalkyl radical onto terminal double bonds, not only the desired linear products but also a certain proportion of branched isomers (with introduction of an additional stereocentre) is formed (cf. Scheme 4).

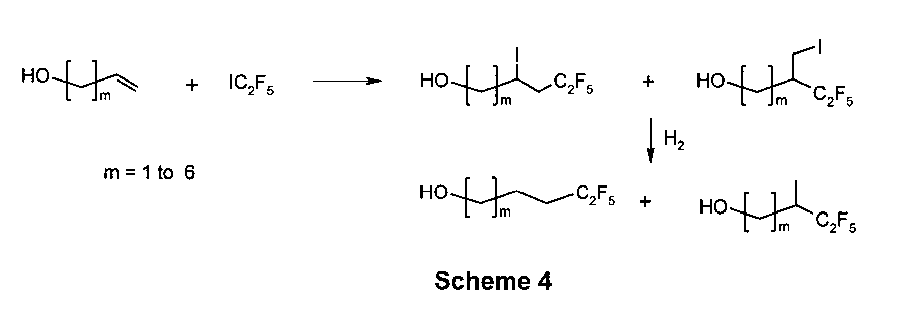

In order to meet the purity requirements needed for the synthesis of active pharmaceutical ingredients, the products of the free-radical addition have to be purified to remove the undesired isomers in the course of the synthesis. This is associated with higher costs and, together with the limited availability (for example of 6-hepten-1-ol (m = 5)), significant problems arise for the conversion of this preparation route to the industrial scale - especially for the preparation of the compound **1.**

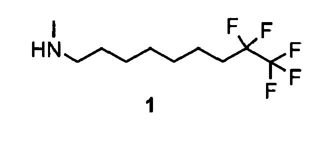

It is therefore an object of the present invention to provide improved processes for preparing α-alkyl(amino)-ω-perfluoro(alkyl)alkanes of the general formula II which dispense with the use of the α-hydroxy-ω-alkenes, which are only of limited availability. It should be possible to use inexpensive, commercially available starting materials. In particular, the starting compounds should be available on the kg scale, i.e. on the industrial scale, such that it is also possible to convert the process according to the invention for preparing the compounds of the general formula II to the industrial scale.

This object is achieved by the process according to the invention for preparing the compounds of the general formula II.

In this process, a Wittig reagent of the general formula V

Hal¹-(CH₂)ₚ-P⁺(Ar)₃(Hal²)⁻ (V)

in which
Hal¹ and Hal² are each independently a halogen atom,
Ar is an aromatic radical, especially a phenyl, o-, m- or p-tolyl radical, and
p is an integer of 3 to 6,
is reacted with 4,4,5,5,5-pentafluoropentanal of the formula VI

H(O)C-(CH₂)₂-C₂F₅ (VI)

in the presence of a very strong base in a Wittig reaction to give an olefin of the general formula VII

Hal¹-(CH₂)₍ₚ₋₁₎-C=C-(CH₂)₂-C₂F₅ (VII)

in which
p is as defined above,
the haloolefin of the general formula VII is coupled to an alkylamine of the general formula VIII

HN(R)(R^{b}) (VIII)

in which
R is as defined in the general formula II and
R^{b} is a hydrogen atom or a benzyl group,
to obtain a compound of the general formula IX

(R^{b})(R)N-(CH₂)₍ₚ₋₁₎-C=C-(CH₂)₂-C₂F₅ (IX)

and
then the double bond is hydrogenated (when R^{b} is a benzyl group with additional elimination of the benzyl group) to obtain a compound of the general formula II

H(R)N-(CH₂)_{¡}-C₂F₅ (II)

in which
R and i are each as defined above.

The Wittig reagents of the formula (V) used are compounds having 3 to 6 carbon atoms in the alkyl moiety (CH₂)ₚ.

The 4,4,5,5,5-pentafluoropentanal of the formula VI to be used as a further starting material is available by known processes from 4,4,5,5,5-pentafluoropentanol.

The compounds of the general formulas VII and IX as well as 8,8,9,9,9-Pentafluoronon-4-en-1-ol, 8,8,9,9,9-Pentafluoronon-4-enyl toluene-4-sulphonate and 8,8,9,9,9-pentafluoronon-4-enyl methanesulphonate exist as the E and Z isomer as well as any mixture of the E and Z isomers.

In the compounds of general formulas I and III R^{17α} and R^{17β} are in particular methyl, ethyl, *n*-propyl, *n*-butyl, *iso*-butyl and *tert.*-butyl, whereby R^{17β} in addition can also be hydrogen, acetyl, propionyl and butanoyl and whereby in this case, the corresponding isomers can be included. In addition, R^{17α} can be ethinyl, 1-propinyl, 2-propinyl, 1-butinyl, 2-butinyl and 3-butinyl as well as as well as trifluoromethl, pentafluoroethyl, heptafluoropropyl and nonafluorobutyl, whereby in this case, the corresponding isomers are also included. R^{17β} is in particular hydrogen, CH₃ or CH₃C(O)-. R^{17α} preferably stands for methyl, ethinyl and trifluoromethyl. In one embodiment of the invention, R in the particular compounds is a methyl group.

In a further embodiment of the invention, i in the particular compounds is 7.

A further variant of the invention envisages that Hal¹ in the particular compounds is a chlorine atom.

Moreover, Hal¹ in the particular compounds may be a bromine or iodine atom.

Ar in the particular compounds is primarily a phenyl radical.

The strong base used may, for example, be potassium *tert*-butoxide, n-butyllithium or lithium trimethylsilylamide.

The present invention provides a process for synthesizing compounds of the general formula II which permits these compounds to be prepared in an efficient manner based on readily available bulk chemicals.

Using this synthesis compounds II are obtained in isomerically pure form, such that they can be used directly and without further purification in the reaction for reaction with a compound of the general formula III to obtain a corresponding compound of the general formula I. The reactions can be performed equally on the laboratory scale and on the industrial scale.

The present invention therefore also relates to a process for preparing compounds of the general formula I 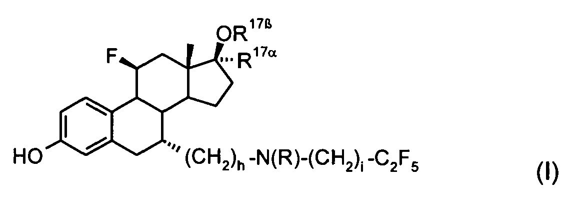 in which
R^{17α} is an alkyl group having 1 to 4 carbon atoms and which may be partially or completely fluorinated or an alkynyl group having 2 to 4 carbon atoms
R^{17β} is an hydrogen atom, an alkyl group having 1 to 4 carbon atoms or an alkanoyl
group having 1 to 4 carbon atoms,
h is an integer of 1 to 6
R is an alkyl group having 1 to 3 carbon atoms and
i is an integer of 6 to 9,
on the industrial scale, in which a compound of the general formula II prepared as above, i.e. according to one of Claims 1 to 7, optionally without isolation from the reaction mixture, is reacted directly with a compound of the general formula III in a manner known per se to give a compound of the general formula I.

The reaction itself is effected analogously to the manner already described, for example, in WO 03/045972 for analogous compounds (process variant 2.2, page 27).

According to the invention, compounds of the general formula II **(1a-d)** are prepared as shown in Scheme 5:

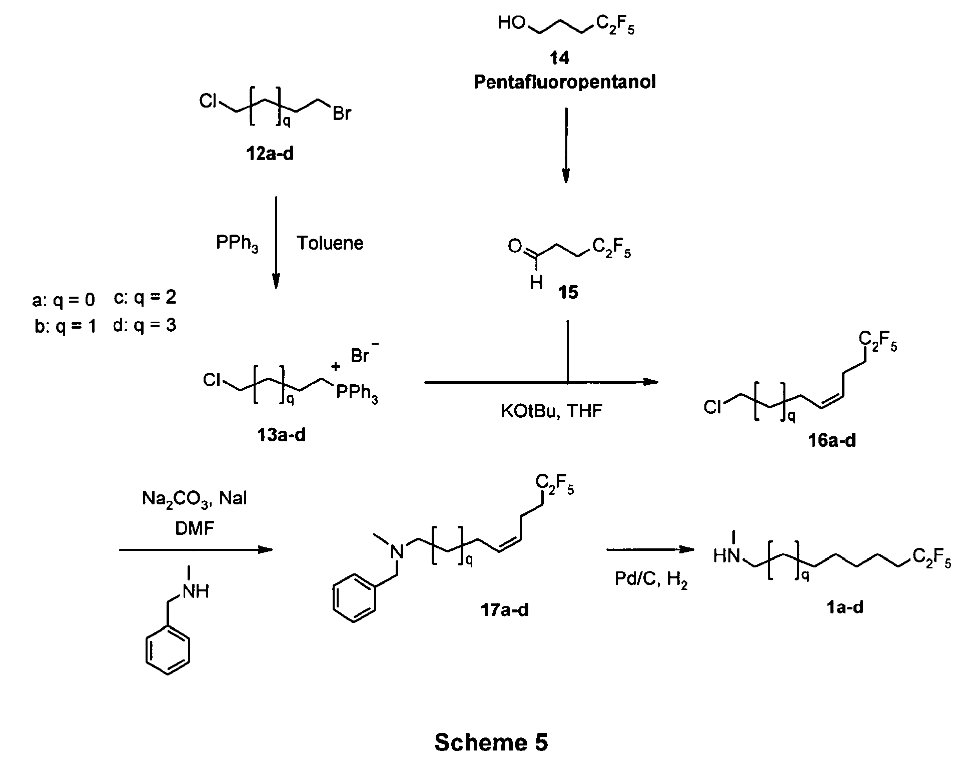

In addition to this synthesis route (use of N-methylbenzylamine), it is equally possible to use N-methylamine to alkylate the α-chloro-ω-perfluoro(alkyl)alkanes **16:**

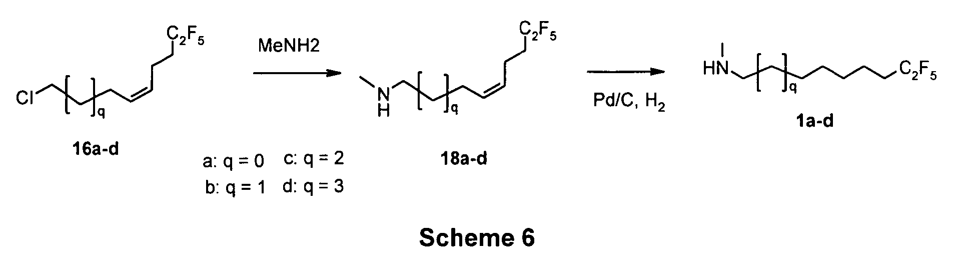

Specifically for the preparation of the series of the α-functionalized ω-pentafluoro-nonanes, it is possible to prepare the compound **24** via the following synthesis route, likewise proceeding from 4,4,5,5,5-pentafluoropentan-1-ol 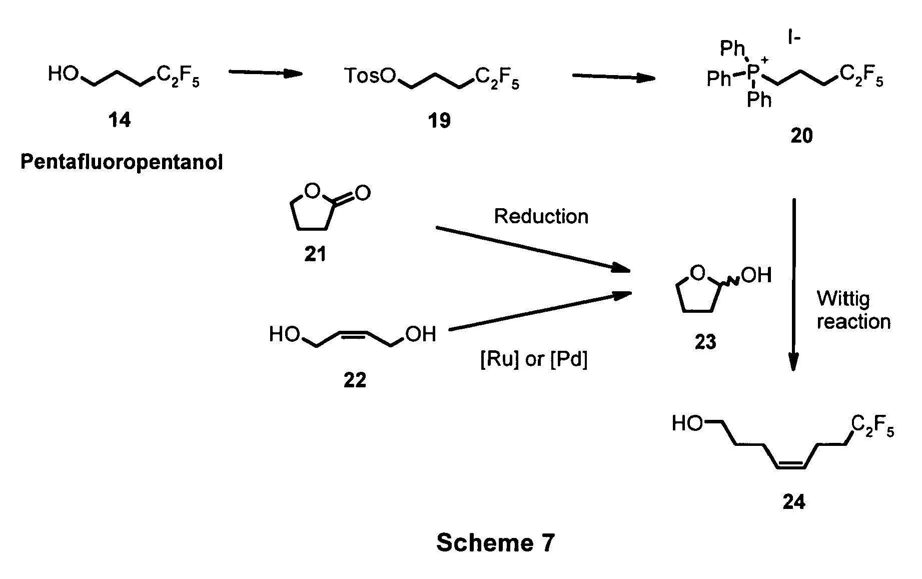 4,4,5,5,5-Pentafluoropentan-1-ol **14** is converted to the tosylate **19** by a known process. The preparation of the novel phosphonium salt **20** may readily be achieved by reacting **19** with sodium iodide and triphenylphosphine in one process step. The tetrahydrofuran-2-ol **23** is prepared by literature processes, either by reduction of butyrolactone **21** or by metal-catalysed double bond isomerization of 1,4-butenediol **22.** Reaction of **23** with **20** in a Wittig reaction affords the novel alcohol 8,8,9,9,9-pentafluoronon-4-en-1-ol **24.**
The hydroxyl group in the compound **24** can be exchanged for a better leaving group by methods familiar to those skilled in the art, for example for a chlorine, bromine or iodine atom or a mesyl or tosyl group. The resulting compounds can then be converted as in Schemes 5 and 6 or as in the Overview Scheme 8 shown below to the compound **1** as a representative of a compound of the general formula II.

In summary, a synthesis of the α-alkyl(amino)-ω-perfluoro(alkyl)alkane **1** is thus possible via different synthesis routes - these are shown once again in the overview scheme below.

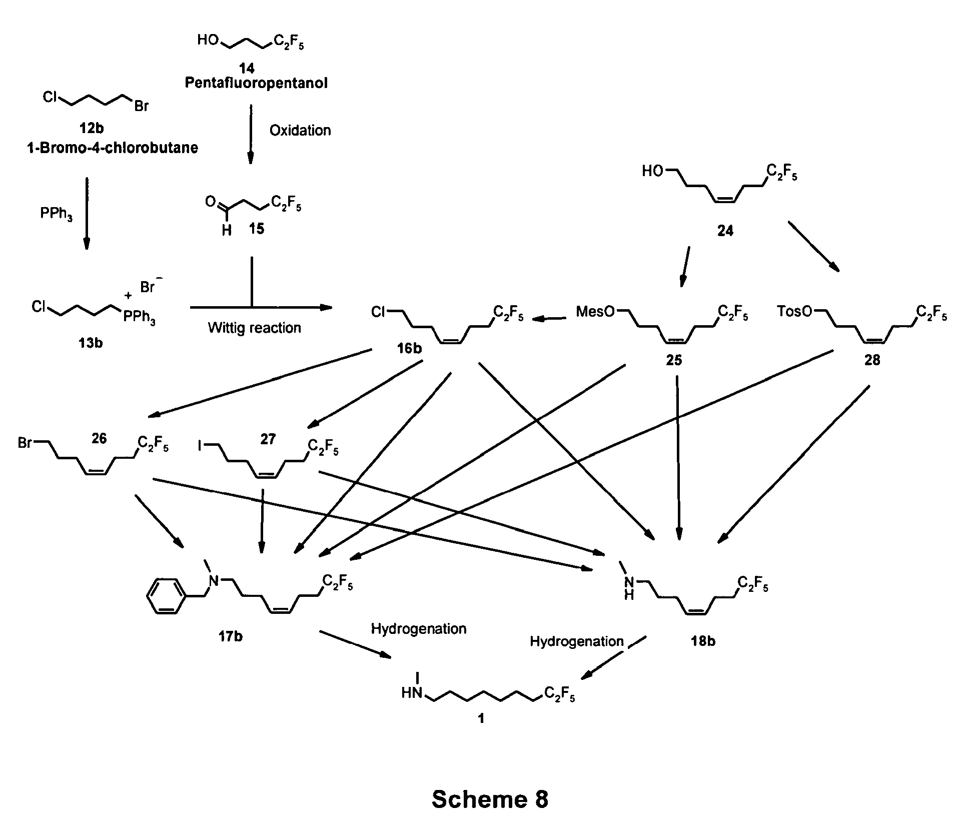

All synthesis routes are based on the use of 4,4,5,5,5-pentafluoropentanol as the starting material, which is supplied in relatively large amounts by several suppliers.

The intermediates of the general formulae II, VII and IX described and the individual compounds 16b, 20, 24, 25, 26, 27 and 28 are novel.

They therefore all belong within the scope of the present invention. Their use in particular for preparing antiestrogens, especially those of the general formula I, likewise forms part of the subject-matter of the present invention.

The examples below are used for a more detailed explanation of the invention.

### Examples

### (4-Chlorobutyl)triphenylphosphonium bromide was prepared analogously to the literature method [ ⁱⁱ ].

4,4,5,5,5-Pentafluoropentan-1-al (or related compounds) has already been described in the literature [see, for example, ⁱⁱⁱ or ^{iv}]. Here, 4,4,5,5,5-pentafluoropentan-1-al was prepared by oxidizing 4,4,5,5,5-pentafluoropentan-1-ol under standard conditions (a dichloromethane solution was prepared by reacting with pyridinium dichromate or else by a TEMPO oxidation [see, for example, ^{v}] - the dichloromethane solution of the 4,4,5,5,5-pentafluoropentan-1-al was used directly in the Wittig reaction owing to the low boiling point). A Swern oxidation of pentafluoropentanol is problematic [ ^{vi} ].

Tetrahydrofuran-2-ol was prepared analogously to the literature method by reduction of butyrolactone or metal-catalysed double bond isomerization of 1,4-butenediol [ ^{vii} ]. 4,4,5,5,5-Pentafluoropentyl toluene-4-sulphonate has already been described in the patent literature [ ^{viii} ].

### Example 1:

### Preparation of 1-chloro-8,8,9,9,9-pentafluoronon-4-ene (16b) (E/Z mixture)

1072.1 g of (4-chlorobutyl)triphenylphosphonium bromide are initially charged in 1200 ml of THF. With cooling to -25°C, a solution of 252.5 g of KOtBu in 1900 ml of THF is added slowly, such that the internal temperature does not rise above -20°C. After the addition has ended, stirring is continued at this temperature for 30 min to complete the deprotonation and then (likewise at a temperature of not more than -20°C) approx. 390 g of 4,4,5,5,5-pentafluoropentan-1-al dissolved in 2000 ml of dichloromethane are added slowly. After the addition has ended, the mixture is first stirred under cold conditions for a further hour, and the reaction mixture is then warmed to room temperature by removing the cold bath.
For workup, the reaction mixture is first concentrated to a residual volume of about 2000 ml under reduced pressure, and then 3800 ml of cyclohexane are added. The suspension thus obtained is filtered through a plug of 1200 g of silica gel, and the solution of the crude product is freed from the solvent under reduced pressure. The residue is finally distilled under reduced pressure at approx. 3-5 mbar and 75-78°C. 210.6 g (34% of theory) of 1-chloro-8,8,9,9,9-pentafluoronon-4-ene (E/Z mixture) are obtained as a colourless liquid.
1 H NMR (400MHz; CDCl₃): 1.80-1.95 (m; 2H); 2.05-2.35 (m; 4H); 2.35-2.50 (m; 2H); 3.60 (tr, 7.0 Hz; 2H); 5.40-5.55 (m; 2H) ppm.

### Example 2:

### Preparation of benzylmethyl(8,8,9,9,9-pentafluoronon-4-en-1-yl)amine (17b) (E/Z mixture)

3.0 g of sodium iodide are initially charged together with 8.0 g of anhydrous sodium carbonate. Subsequently, a solution of 25.2 g of 1-chloro-8,8,9,9,9-pentafluoronon-4-ene (E/Z mixture) dissolved in 126 ml of DMF is added. After adding 20.2 ml of N-benzylmethylamine, the reaction mixture is heated to an internal temperature of about 70°C to complete the reaction (approx. 7 h).
For workup, 126 ml of methyl tert-butyl ether and 126 ml of water are added. The phases are separated, and the aqueous phase is extracted first 2x with 63 ml each time of methyl tert-butyl ether. Subsequently, the combined organic phases are washed 3x with 63 ml each time of water and then concentrated to dryness. Small amounts of impurities are finally removed by filtration through a plug of 100 g of silica gel. 25 g (70% of theory) of benzylmethyl(8,8,9,9,9-pentafluoronon-4-en-1-yl)amine (E/Z mixture) are obtained in the form of a slightly yellow liquid.
¹H NMR (400MHz; CDCl₃): 1.55-1.70 (m; 2H); 1.95-2.15 (m; 4H); 2.2 (s, 3H); 2.30-2.45 (m; 2H); 3.5 (s, 2H); 5.25- 5.50 (m; 2H); 7.20-7.35 (m; 5H) ppm.

### Example 3:

### Preparation of methyl(8,8,9,9,9-pentafluorononyl)amine (1) (from hydrogenation and debenzylation)

264 g of benzylmethyl(8,8,9,9,9-pentafluoronon-4-en-1-yl)amine (E/Z mixture) are dissolved in 2600 ml of methanol. After the addition of 10.6 g of Pd/C (10%), the reaction mixture is stirred in a hydrogen atmosphere at a pressure of 1 bar until the hydrogen absorption stops.
For workup, the reaction mixture is filtered, the residue is washed with 3x100 ml of methanol and the filtrate is concentrated under reduced pressure. The residue is taken up in 2500 ml of methyl tert-butyl ether and 2500 ml of water. With ice bath cooling, 250 ml of NaOH (50%) are used to establish a pH of > 12. The phases are separated. The aqueous phase is extracted 3x with 250 ml each time of MTBE. The combined organic phases are washed 3x with 250 ml each time of water and then concentrated. 169.6 g (87% of theory) of methyl(8,8,9,9,9-pentafluorononyl)amine are obtained as a colourless liquid. Small amounts of impurities can be removed by distillation under reduced pressure (at 4 mbar and 90°C).
¹H NMR (400MHz; CDCl₃): 1.1 (br s; NH); 1.3-1.7 (m; 10H); 1.9-2.1 (m; 2H); 2.4 (s; 3H); 2.6 (tr; 7.2 Hz; 2H) ppm.

### Example 4:

### Preparation of methyl(8,8,9,9,9-pentafluoronon-4-en-1-yl)amine (18b) (E/Z mixture)

5 g of 1-chloro-8,8,9,9,9-pentafluoronon-4-ene (E/Z mixture) are dissolved in 25 ml of ethanol and then admixed with 60 ml of aqueous methylamine solution (40% strength) and heated at jacket temperature 80°C in an autoclave over a period of 16 h. After cooling to rt, 50 ml of MTBE and 20 ml of water are added, and the phases are separated. The aqueous phase is reextracted 2x with 30 ml of MTBE and washed with 20 ml of NaOH (1 molar). The organic phase is then concentrated to 10 ml on a rotary evaporator and adjusted to pH <2 with approx. 10 ml of 10% sulphuric acid. The aqueous product solution is washed with 15 ml of hexane and then 2x with 20 ml each time of a mixture of hexane and MTBE (3:1). Addition of 28 ml of a 1 molar NaOH solution adjusts the pH of the aqueous phase to >12, and the product is extracted 3 times with MTBE. The combined organic phases are concentrated to give 3.35 g (68% of theory) of methyl(8,8,9,9,9-pentafluoronon-4-en-1-yl)amine (E/Z mixture) as an orange oil.
¹H NMR (400 MHz, CDCl₃): 1.1 (br s; NH); 1.6 (quintet; 7.3 Hz; 2H); 2.0-2.2 (m; 4H); 2.3-2.4 (m; 2H); 2.5 (s; 3H); 2.6 (t; 7.3 Hz; 2 H); 5.3-5.6 (m; 2H) ppm.

### Example 5:

### Preparation of methyl(8,8,9,9,9-pentafluorononyl)amine (1) (from hydrogenation)

1.5 g of methyl(8,8,9,9,9-pentafluoronon-4-en-1-yl)amine (E/Z mixture) are dissolved in 15 ml of MTBE and admixed with 1.5 ml of acetic acid. After the addition of 80 mg of Pd/C (10%), the reaction mixture is stirred in a hydrogen atmosphere at a pressure of 1 bar until the hydrogen absorption stops. For workup, the reaction mixture is filtered, the residue is washed and 15 ml of water are added. Thereafter, the pH is adjusted to >12 with approx. 2 ml of 50% NaOH and the phases are separated. The aqueous phase is then reextracted 2x with 7.5 ml each time of MTBE and the combined organic phases are reextracted with 7.5 ml of water. The solvent is removed to give 1.42 g (92% of theory) of methyl(8,8,9,9,9-pentafluorononyl)amine as a slightly yellow oil.
¹H NMR (400MHz; CDCl₃): 1.1 (br s; NH); 1.3-1.7 (m; 10H); 1.9-2.1 (m; 2H); 2.4 (s; 3H); 2.6 (tr; 7.2 Hz; 2H) ppm.

### Example 6:

### Preparation of (4,4,5,5,5-pentafluoropentyl)triphenylphosphonium iodide (20)

100 g of 4,4,5,5,5-pentafluoropentyl toluene-4-sulphonate are dissolved in 300 ml of acetonitrile and mixed with 86.8 g of triphenylphosphine and 49.6 g of sodium iodide. The suspension is heated to 90°C over a period of 8 h. Thereafter, the solid is filtered off, and the residue is washed twice with 300 ml of acetonitrile. The filtrate is redistilled into approx. 600 ml of ethyl acetate, and the product precipitates out as a solid. The crystals are filtered off, washed with 200 ml of ethyl acetate and dried in a drying cabinet. 151 g of pale yellow crystals are obtained.
¹H NMR (400MHz; DMSO-d₆): 1.70-1.80 (m; 2H); 2.35-2.55 (m; 2H); 3.65-3.75 (m; 2H); 7.70-7.95 (m; 15H) ppm.

### Example 7:

### Preparation of 8,8,9,9,9-pentafluoronon-4-en-1-ol (24) (E/Z mixture)

2.97 g of (4,4,5,5,5-pentafluoropentyl)triphenylphosphonium iodide are suspended in 2.4 ml of THF and then under cold conditions 665 mg of KOtBu dissolved in 2.4 ml of THF are added. After 30 min, a solution of 570 mg of tetrahydrofuran-2-ol in 1.2 ml of THF is added dropwise, and the mixture is stirred under cold conditions for a further 30 min and then warmed to room temperature over a period of 3 h. The reaction is quenched by adding 5 ml of water, and organic phase is removed after adding 10 ml of MTBE. The aqueous phase is then reextracted 2x with 5 ml each time of MTBE, and the organic phase is concentrated under reduced pressure. The crude substance is admixed with 10 ml of hexane, the solid is filtered off with suction and the filtercake is washed with 10 ml of hexane. Subsequently, the mixture is concentrated on a rotary evaporator and the product is chromatographed on silica gel. 830 mg (66% of theory) of 8,8,9,9,9-pentafluoronon-4-en-1-ol (E/Z mixture) are obtained as a colourless oil.
¹H NMR (400MHz; CDCl₃): 1.35 (br s; OH); 1.65 (quintet; 7.0 Hz; 2H); 2.0-2.2 (m; 4H); 2.3-2.4 (m; 2H); 3.65 (tr; 7.0 Hz; 2H); 5.3-5.55 (m; 2H) ppm.

### Example 8:

### Preparation of 1-bromo-8,8,9,9,9-pentafluoronon-4-ene (26) (E/Z mixture)

5 g of 1-chloro-8,8,9,9,9-pentafluoronon-4-ene (E/Z mixture) are suspended with 10.26 g of sodium bromide in 25 ml of DMF and then heated to 130°C over a period of 5 h. After adding 30 ml of ethyl acetate and 50 ml of water, the phases are separated, and the organic phase is then washed four times with 50 ml each time of water and dried over sodium sulphate. The solvent is removed and the crude product is chromatographed on silica gel. 4.33 g of a colourless oil are obtained.
¹H NMR (400MHz; CDCl₃): 1.85-2.00 (m; 2H); 2.05-2.20 (m; 4H); 2.20-2.40 (m; 2H); 3.40 (tr; 7.0 Hz; 2H); 5.35-5.50 (m; 2H) ppm.

### Example 9:

### Preparation of 1-iodo-8,8,9,9,9-pentafluoronon-4-ene (27) (E/Z mixture)

10 g of 1-chloro-8,8,9,9,9-pentafluoronon-4-ene (E/Z mixture) are suspended with 33.12 g of potassium iodide in 50 ml of DMF and stirred at 130°C over a period of 2 h. For workup of the reaction mixture, 75 ml of hexane and 50 ml of water are added, and the phases are separated. The aqueous phase is extracted once more with hexane and the combined organic phases are washed twice with 50 and 25 ml of water. The solvent is removed and the crude product is chromatographed on silica gel. 8.47 g of a colourless oil are obtained.
¹H NMR (400MHz; CDCl₃): 1.80-1.95 (m; 2H); 2.00-2.25 (m; 4H); 2.30-2.45 (m; 2H); 3.20 (tr, 7.0 Hz; 2H); 5.35-5.50 (m; 2H) ppm.

### Example 10:

### Preparation of 8,8,9,9,9-pentafluoronon-4-enyl methanesulphonate (25) (E/Z mixture)

100 mg 8,8,9,9,9-pentafluoronon-4-en-1-ol are dissolved in 2.8 ml of dichloromethane. Then, 0.17 ml triethyl amine and 107 mg methanesulfonic acid chloride are added and the reaction mixture is stirred over a period of 18 h at room temperature. 7.2 ml dichloromethane are added and the reaction mixture is extracted with 5 ml water and 10 ml of brine solution. The organic phase is dried with sodium sulphate and the organic solvent evaporated yielding 110 mg of a pale yellow oil.
¹H-NMR (400MHz; CDCl₃): 1.75-1.90 (m; 2H); 2.00-2.25 (m; 4H); 2.30-2.40 (m; 2H); 3.00 (s; 3H); 4.25 (tr; 6.4 Hz; 2H); 5.35-5.50 (m; 2H) ppm.

### Example 11:

### Preparation of 8,8,9,9,9-pentafluoronon-4-enyl toluene-4-sulphonate (28) (E/Z mixture)

100 mg 8,8,9,9,9-pentafluoronon-4-en-1-ol are dissolved in 2.8 ml of dichloromethane. Then, 0.17 ml triethyl amine and 107 mg toluene-4-sulfonic acid chloride are added and the reaction mixture is stirred over a period of 18 h at room temperature. 7.2 ml dichloromethane are added and the reaction mixture is extracted with 5 ml water and 10 ml of brine solution. The organic phase is dried with sodium sulphate and the organic solvent evaporated yielding 156 mg of a pale yellow oil.
¹H-NMR (400MHz; CDCl₃): 1.65-1.75 (m; 2H); 1.95-2.15 (m; 4H); 2.20-2.30 (m; 2H); 2.45 (s; 3H); 4.05 (tr; 7.0 Hz; 2H); 5.30-5.45 (m; 2H); 7.35 (d; 8.1 Hz; 2H) 7.80 (d; 8.1 Hz; 2H) ppm.

### Literature

ⁱ J. D. Park, F. E. Rogers, J. R. Lacher, J. Org. Chem. 1961, 26, 2089 - 2095.
ⁱⁱ W.H. Pearson, K-C. Lin, Tetrahedron Lett. 1990, 31, 7571 - 7574.
ⁱⁱⁱ I. Ojima, K. Kato, M. Okabe, T. Fuchikami, J. Amer. Chem. Soc. 1987, 109, 7714 - 7720.
^{iv} a) G. Pozzi, S. Quici, I. Shepperson, Tetrahedron Lett. 2002, 43, 6141 - 6144. b) C. Rocaboy, W. Bauer, J.A. Gladysz Eur. J. Org. Chem. 2000, 65, 2621 - 2628.
^{v} Organic Syntheses Col. Vol. 8, 367.
^{vi} L. Leveque, M. Le Blanc, R. Pastor, Tetrahedron Lett. 1998, 39, 8857 - 8860.
^{vii} a) K. Watanabe, Yuto Suzuki, K. Akoi, A. Sakakura, Kiyotake Suenaga, H. Kigoshi, J. Org. Chem. 2004, 69, 7802-7808 b) B. Seddig, J. Alm, Journal f. prakt. Chemie 1987, 329, 711-716.
^{viii} Chugai Seiyaku Kabushiki Kaisha, US6645951 B1 (2003/11/11), Appl. US2001-7-19608 (2001/07/16)

## Claims

1. Process for preparing α-alkyl(amino)-ω-(perfluoroalkyl)alkanes of the general formula II
H(R)N-(CH₂)ᵢ-C₂F₅ (II)
in which
R is an alkyl group having 1 to 3 carbon atoms and
i is an integer of 6 to 9,
**characterized in that** a Wittig reagent of the general formula V
Hal¹-(CH₂)ₚ-P⁺(Ar)₃(Hal²)⁻ (V)
in which
Hal¹ and Hal² are each independently a halogen atom,
Ar is an aromatic radical, especially a phenyl, o-, m- or p-tolyl radical, and
p is an integer of 3 to 6,
is reacted with 4,4,5,5,5-pentafluoropentanal of the formula VI
H(O)C-(CH₂)₂-C₂F₅ (VI)
in the presence of a strong base in a Wittig reaction to give an olefin of the general formula VII
Hal¹-(CH₂)₍ₚ₋₁₎-C=C-(CH₂)₂-C₂F₅ (VII)
in which
p is as defined above,
the haloolefin of the general formula VII is coupled to an alkylamine of the general formula VIII
HN(R)(R^{b}) (VIII)
in which
R is as defined in the general formula II and
R^{b} is a hydrogen atom or benzyl group,
to obtain a compound of the general formula IX
(R^{b})(R)N-(CH₂)₍ₚ₋₁₎-C=C-(CH₂)₂-C₂F₅ (IX)
and
then the double bond is hydrogenated (when R^{b} is a benzyl group with elimination of the benzyl group) to obtain a compound of the general formula II
H(R)N-(CH₂)ᵢ-C₂F₅ (II)
in which
R and i are each as defined above.

2. Process according to Claim 1, in which R in the particular compounds is a methyl group.

3. Process according to Claim 1 or 2, in which i in the particular compounds is 7.

4. Process according to one of the preceding claims, in which Hal¹ in the particular compounds is a chlorine atom.

5. Process according to one of the preceding claims, in which Hal¹ in the particular compounds is a bromine or iodine atom.

6. Process according to one of the preceding claims, in which Ar in the particular compounds is a phenyl radical.

7. Process according to one of the preceding claims, in which the strong base is potassium *tert*-butoxide, n-butyllithium or lithium trimethylsilylamide.

8. Compounds of the general formula VII
Hal¹-(CH₂)₍ₚ₋₁₎-C=C-(CH₂)₂-C₂F₅ (VII)
in which
Hal¹ and p are each as defined in Claim 1.

9. a) 1-Chloro-8,8,9,9,9-pentafluoronon-4-ene (E and Z isomer as well as any mixture of E and Z isomer) according to Claim 8
b) 1-Bromo-8,8,9,9,9-pentafluoronon-4-ene (E and Z isomer as well as any mixture of E and Z isomer) according to Claim 8
c) 1-lodo-8,8,9,9,9-pentafluoronon-4-ene (E and Z isomer as well as any mixture of E and Z isomer) according to Claim 8.

10. Compounds of the general formula VII according to Claim 8 or 9 for use as intermediates for synthesis of antiestrogens of the general formula I.

11. Compounds of the general formula IX
(R^{b})(R)N-(CH₂)₍ₚ₋₁₎-C=C-(CH₂)₂-C₂F₅ (IX)
in which
R and R^{b} and also p are each as defined in Claim 1.

12. Benzylmethyl(8,8,9,9,9-pentafluoronon-4-en-1-yl)amine (E and Z isomer as well as any mixture of E and Z isomer) according to Claim 11.

13. Methyl(8,8,9,9,9-pentafluoronon-4-en-1-yl)amine (E and Z isomer as well as any mixture of E and Z isomer) according to Claim 11.

14. Compounds of the general formula IX according to Claim 11, 12 or 13 for use as intermediates for synthesis of antiestrogens of the general formula I.

15. Compounds of the general formula II
H(R)N-(CH₂)ᵢ-C₂F₅ (II)
in which
R is an alkyl group having 1 to 3 carbon atoms and
i is an integer of 6 to 9.

16. Methyl(8,8,9,9,9-pentafluoronon-1-yl)amine according to Claim 15.

17. Compounds of the general formula II according to Claim 15 or 16 for use as intermediates for synthesis of antiestrogens of the general formula I.

18. (4,4,5,5,5-Pentafluoropentyl)triphenylphosphonium iodide.

19. (4,4,5,5,5-Pentafluoropentyl)triphenylphosphonium iodide according to Claim 18 for use as an intermediate for synthesis of antiestrogens of the general formula I.

20. 8,8,9,9,9-Pentafluoronon-4-en-1-ol (E and Z isomer as well as any mixture of E and Z isomer).

21. 8,8,9,9,9-Pentafluoronon-4-en-1-ol (E and Z isomer as well as any mixture of E and Z isomer) according to Claim 20 for use as an intermediate for synthesis of antiestrogens of the general formula I.

22. a) 8,8,9,9,9-Pentafluoronon-4-enyl toluene-4-sulphonate (E and Z isomer as well as any mixture of E and Z isomer)
b) 8,8,9,9,9-pentafluoronon-4-enyl methanesulphonate (E and Z isomer as well as any mixture of E and Z isomer).

23. a) 8,8,9,9,9-Pentafluoronon-4-enyl toluene-4-sulphonate (E and Z isomer as well as any mixture of E and Z isomer) and
b) 8,8,9,9,9-pentafluoronon-4-enyl methanesulphonate (E and Z isomer as well as any mixture of E and Z isomer) according to Claim 22 for use as an intermediate for synthesis of antiestrogens of the general formula I.

24. Process for preparing compounds of the general formula I 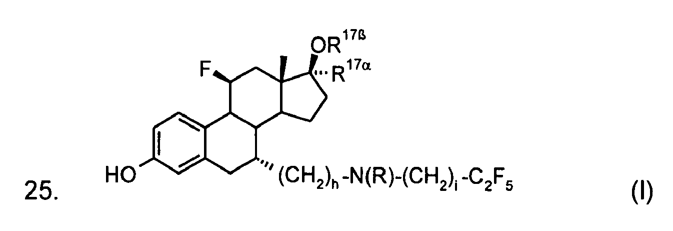 in which
R^{17α} is an alkyl group having 1 to 4 carbon atoms and which may be partially or completely fluorinated or an alkynyl group having 2 to 4 carbon atoms
R^{17β} is an hydrogen atom, an alkyl group having 1 to 4 carbon atoms or an alkanoyl group having 1 to 4 carbon atoms,
h is an integer of 1 to 6
R is an alkyl group having 1 to 3 carbon atoms and
i is an integer of 6 to 9,
on the industrial scale, **characterized in that** a compound of the general formula II prepared according to one of Claims 1 to 7, optionally without isolation from the reaction mixture, is reacted directly with a compound of the general formula III in a manner known per se to give a compound of the general formula I.
